# EUROPEAN PATENT APPLICATION

(11) **EP 4 850 666 A1**
(43) Date of publication of application: **09.12.2026**
(21) Application number: 25887443.7
(22) Date of filing: 09.09.2025
(51) Int. Cl.: A61K 8/92, A61K 8/85, A61K 8/49, A61K 8/44, A61K 8/06

(54) **OIL-IN-WATER TYPE COSMETIC COMPOSITION FOR BLOCKING ULTRAVIOLET RAYS, AND PREPARATION METHOD THEREFOR**

(30) Priority: 15.04.2025 KR 20250049141
(71) Applicant: Cosmax, Inc., Hwaseong-si, Gyeonggi-do 18622 (KR)
(72) Inventor: KIM, Sun Young, Seongnam-si, Gyeonggi-do 13486 (KR); LIM, Hyun Ju, Seongnam-si, Gyeonggi-do 13486 (KR); PARK, Chun Ho, Seongnam-si, Gyeonggi-do 13486 (KR)
(74) Representative: Croce, Valeria
(86) International application number: PCT/KR2025/013969
(87) International publication number: WO 2026/221171

(57) **Abstract**

The present disclosure relates to an oil-in-water type sunscreen cosmetic composition, wherein, according to an aspect, the composition may include a small amount of ultraviolet-blocking components that cause heavy feeling of use, a whitening phenomenon, or greasiness, thereby improving the feeling of use while exhibiting high ultraviolet-blocking efficacy and excellent long-term water resistance, and thus may be usefully employed as an oil-in-water type waterproof sunscreen cosmetic composition.

## Description

### Technical Field

The present disclosure relates to an oil-in-water type sunscreen cosmetic composition and a method of producing the same.

### Background Art

Ultraviolet (UV) radiation is a factor that has a very harmful effect on skin health, causing various types of skin damage by penetrating into the dermis layer to destroy structural proteins of the skin such as collagen and elastin, thereby inducing wrinkles and loss of elasticity, or by directly damaging DNA in the epidermis layer to promote inflammatory responses and pigmentation. In particular, prolonged exposure to UV radiation increases the generation of reactive oxygen species (ROS), which induces oxidative stress, thereby accelerating the aging of skin cells and, in severe cases, leading to skin cancer.

Recently, due to environmental issues such as global warming and destruction of the ozone layer, the UV index has been continuously increasing, and the necessity of year-round protection against UV radiation has been further emphasized. In this situation, it is essential to use waterproof-type sunscreens that are not easily removed by sweat or water; however, existing waterproof sunscreens include oil in the outer phase to enhance adhesion to the skin. However, such water-in-oil type sunscreens exhibit an unpleasant and heavy feeling of use, such as making the skin feel stuffy and sticky, and they are not easily removed during facial cleansing, and thus may clog pores and cause skin trouble. In addition, oil-in-water type sunscreens have a problem in that their waterproof effect may be reduced.

Meanwhile, if the content of UV-blocking components is increased for effective UV blocking, a pronounced whitening phenomenon of the formulation occurs, causing the skin tone to appear unnatural, and the heavy and sticky feeling of use is further aggravated. Such whitening phenomenon and heavy feeling of use reduce the frequency and amount of sunscreen application by consumers, thereby ultimately preventing sufficient UV blocking from being achieved. Accordingly, there is a need for an innovative formulation technology capable of maintaining excellent blocking effects while adjusting the content of UV-blocking components to an appropriate level.

Therefore, there is a need to develop a novel sunscreen cosmetic composition capable of maintaining excellent UV-blocking effects and waterproof performance, while optimizing the content of UV-blocking components to minimize the whitening phenomenon and provide a refreshing feeling of use on the skin.

Accordingly, the inventors of the present disclosure developed an oil-in-water type waterproof sunscreen cosmetic composition by adjusting the content of UV-blocking components, and completed the present disclosure by confirming that the composition exhibits excellent feeling of use, moisture sensation, long-term water resistance, and UV-blocking efficacy.

### Disclosure of Invention

### Technical Problem

One aspect is to provide a sunscreen cosmetic composition including a film former and an ultraviolet (UV)-blocking agent.

Another aspect is to provide a use of a composition including a film former and an UV-blocking agent for the preparation of a sunscreen cosmetic composition.

Another aspect is to provide a method of protecting the skin of an individual from UV radiation, including applying an effective amount of a composition including a film former and an UV-blocking agent to the individual.

### Solution to Problem

One aspect provides a sunscreen cosmetic composition including a film former and an ultraviolet (UV)-blocking agent.

The composition may be in any formulation commonly used in cosmetic compositions, but specifically may be of an oil-in-water (O/W) type.

The composition may include the film former and the UV-blocking agent in an oil phase portion.

The film former may include a hydrogenated castor oil/sebacic acid copolymer, and may further include one or more selected from the group consisting of a capryloyl glycerin/sebacic acid copolymer, and a castor oil/IPDI copolymer, but is not limited thereto.

The hydrogenated castor oil/sebacic acid copolymer refers to a synthetic polymer compound produced by polymerizing hydrogenated castor oil and sebacic acid. The hydrogenated castor oil/sebacic acid copolymer is formed through an esterification reaction between a hydrogenated derivative of ricinoleic acid derived from castor oil and sebacic acid, a dicarboxylic acid, and has excellent biocompatibility as a fatty acid-derived component.

Since the composition may improve the UV-blocking effect by including the hydrogenated castor oil/sebacic acid copolymer as the film former, thereby reducing the content of the UV-blocking component. Accordingly, the composition may have an excellent feeling of use while including a small amount of the UV-blocking component, and at the same time exhibit excellent UV-blocking effect and long-term water resistance.

The hydrogenated castor oil/sebacic acid copolymer may be included in an amount of 0.001 to 30 wt%, for example, 0.001 to 20 wt%, 0.001 to 10 wt%, 0.01 to 10 wt%, 0.001 to 5 wt%, 0.01 to 5 wt%, 0.1 to 5 wt%, 0.001 to 2.4 wt%, 0.01 to 2.4 wt%, 0.05 to 2.4 wt%, 0.1 to 2.4 wt%, 0.001 to 2.3 wt%, 0.01 to 2.3 wt%, 0.05 to 2.3 wt%, 0.1 to 2.3 wt%, 0.01 to 2 wt%, 0.05 to 2 wt%, 0.1 to 2 wt%, or 0.5 to 2 wt%, based on the total weight of the composition. When outside the range, the formulation of the composition may not be formed, or problems such as deterioration in spreadability, water resistance, or UV-blocking effect may occur.

The UV-blocking agent may include an organic or inorganic UV-blocking agent, and specifically may include one or more selected from the group consisting of ethylhexyl triazone and diethylamino hydroxybenzoyl hexyl benzoate, and may further include one or more selected from the group consisting of zinc oxide, titanium dioxide, ethylhexyl triazone, bis-ethylhexyloxyphenol methoxyphenyl triazine, diethylamino hydroxybenzoyl hexyl benzoate, drometrizole trisiloxane, ethylhexyl methoxycinnamate, octocrylene, homosalate, butyl methoxydibenzoylmethane, ethylhexyl salicylate, methylene bis-benzotriazolyl tetramethylbutylphenol, diethylhexyl butamido triazone, phenylbenzimidazole sulfonic acid, tris-biphenyl triazine, and terephthalylidene dicamphor sulfonic acid, but is not limited thereto.

According to an example, the composition may include ethylhexyl triazone or diethylamino hydroxybenzoyl hexyl benzoate, and it was confirmed that the composition exhibits excellent UV-blocking effect, long-term water resistance, and feeling of use by including hydrogenated castor oil/sebacic acid copolymer as the film former. In addition, it was confirmed that when the composition further includes ethylhexyl triazone and diethylamino hydroxybenzoyl hexyl benzoate in addition to the hydrogenated castor oil/sebacic acid copolymer, the UV-blocking effect, long-term water resistance, and feeling of use are particularly excellent.

The ethylhexyl triazone may be included in an amount of 0.001 to 30 wt%, for example 0.001 to 20 wt%, 0.05 to 20 wt%, 0.01 to 20 wt%, 0.01 to 10 wt%, 0.1 to 10 wt%, 0.01 to 7 wt%, 0.1 to 7 wt%, 0.01 to 5 wt%, 0.1 to 5 wt%, or 0.5 to 5 wt%, based on the total weight of the composition. When outside the range, the formulation of the composition may not be formed, or problems such as deterioration in spreadability, water resistance, or UV-blocking effect may occur.

The diethylamino hydroxybenzoyl hexyl benzoate may be included in an amount of 0.001 to 30 wt%, for example 0.01 to 30 wt%, 0.05 to 30 wt%, 0.01 to 20 wt%, 0.1 to 20 wt%, 0.01 to 15 wt%, 0.1 to 15 wt%, 0.01 to 10 wt%, 0.1 to 10 wt%, or 0.5 to 10 wt%, based on the total weight of the composition. When outside the range, the formulation of the composition may not be formed, or problems such as deterioration in spreadability, water resistance, or UV-blocking effect may occur.

The composition may include the film former and the UV-blocking agent in a weight ratio of 1:0.01 to 50, for example 1:0.05 to 50, 1:0.1 to 50, 1:0.1 to 40, 1:0.5 to 40, 1:0.1 to 30, or 1:0.5 to 30. When outside the range, the formulation of the composition may not be formed, or problems such as deterioration in spreadability, water resistance, or UV-blocking effect may occur.

The composition may further include one or more aqueous phase components selected from polyols, solvents, preservatives, and surfactants.

The polyol may include one or more selected from propanediol, 1,2-hexanediol, and pentylene glycol, and the polyol may be included in an amount of 0.01 to 20 wt%, for example 0.1 to 15 wt%, 0.5 to 10 wt%, or 0.8 to 6 wt%, based on the total weight of the composition.

The preservative may include hydroxyacetophenone, and the preservative may be included in an amount of 0.001 to 10 wt%, for example 0.01 to 5 wt%, 0.05 to 1 wt%, 0.1 to 0.5 wt%, or 0.3 wt%, based on the total weight of the composition.

The surfactant may include sodium stearoyl glutamate, and the surfactant may be included in an amount of 0.001 to 10 wt%, for example 0.001 to 5 wt%, 0.01 to 0.5 wt%, 0.05 to 0.2 wt%, or 0.1 wt%, based on the total weight of the composition.

The composition may further include one or more oil phase components selected from surfactants, conditioning agents, and higher alcohols.

The surfactant may include a polyglyceryl-based surfactant or a nonionic surfactant.

The polyglyceryl-based surfactant may include one or more selected from polyglyceryl-3 methylglucose distearate, polyglyceryl-3 distearate, polyglyceryl-6 distearate, polyglyceryl-6 stearate, polyglyceryl-6 behenate, polyglyceryl-3 diisostearate, and polyglyceryl-3 stearate, and may be included in an amount of 0.01 to 20 wt%, for example 0.01 to 10 wt%, 0.1 to 5 wt%, 1 to 3 wt%, or 2 wt%, based on the total weight of the composition.

The nonionic surfactant may include glyceryl stearate, and the nonionic surfactant may be included in an amount of 0.001 to 20 wt%, for example 0.001 to 10 wt%, 0.01 to 5 wt%, 0.1 to 1 wt%, or 0.5 wt%, based on the total weight of the composition.

The conditioning agent may include diisopropyl sebacate, and the conditioning agent may be included in an amount of 0.1 to 50 wt%, for example 1 to 40 wt%, 10 to 30 wt%, 15 to 25 wt%, or 20 wt%, based on the total weight of the composition.

The higher alcohol may include behenyl alcohol, and the higher alcohol may be included in an amount of 0.001 to 20 wt%, for example 0.001 to 10 wt%, 0.01 to 5 wt%, 0.1 to 1 wt%, or 0.5 wt%, based on the total weight of the composition.

The composition may further include one or more thickening agent component selected from carbomer, sodium acrylate crosspolymer-2, acrylate/C10-30 alkyl acrylate crosspolymer, and polyacrylate crosspolymer-6.

The thickening agent component may be included in an amount of 0.0001 to 10 wt%, for example 0.001 to 1 wt%, or 0.01 to 0.5 wt%, based on the total weight of the composition.

The composition may be prepared in any formulation that may be typically used in a cosmetic composition, but may be an oil-in-water (O/W) type cosmetic composition including an aqueous phase portion and an oil phase portion.

The cosmetic composition may be a waterproof sunscreen cosmetic composition of oil-in-water type, in which the feeling of use, moisture feel, and water resistance are improved.

The feeling of use refers to the overall sensory and organoleptic characteristics experienced by a user when applied to the skin. The feeling of use may include various factors such as the product's spreadability, absorbability, skin adhesion, residual feeling, moisture feel, moistness, stickiness, freshness, lightness, and heaviness. The feeling of use is determined by the physical properties, chemical composition, and formulation, etc. of the cosmetic, and is an important quality characteristic that directly affects consumer preference and whether or not they will continue to use it.

The feeling of use may refer to whitening phenomenon, stickiness, pore clogging, or skin irritation caused by the inclusion of UV-blocking components.

The moisture feel refers to the moist and hydrated sensory characteristics perceived when applied to the skin. This may be determined by the moisture content, moisture retention capacity, moisture delivery ability, etc. in the composition.

The water resistance refers to the property of withstanding water or moisture. In other words, may refer to a property that maintains its original function and characteristics even when exposed to water, and the higher the water resistance and UV-blocking ability, the better the UV-blocking ability is maintained even under moisture conditions. The water resistance may include long-term water resistance. The water resistance may be used interchangeably with waterproof.

The long-term water resistance refers to the property of withstanding water or moisture for a longer period of time than water resistance. It may refer to the degree to which the composition is not washed off and the UV-blocking ability is maintained even under conditions of prolonged exposure to moisture such as water or sweat. The long-term water resistance may be used interchangeably with waterproof.

The UV blocking refers to all actions of protecting the skin from UV (UV) radiation. The UV blocking may include UVA or UVB blocking.

The oil-in-water type sunscreen cosmetic composition may exhibit excellent UV-blocking effect while including a small amount of UV-blocking components, and at the same time, due to the small content of UV-blocking components, may be a sunscreen cosmetic composition of oil-in-water type in which the whitening phenomenon, stickiness, pore clogging, skin irritation, and skin allergic reactions are improved, together with improved feeling of use, improved water resistance and long-term water resistance, and improved skin moisturizing effect.

In an example, it was confirmed that an oil-in-water type sunscreen cosmetic composition including 0.5 to 2 wt% of hydrogenated castor oil/sebacic acid copolymer as the film former, based on the total weight of the composition, exhibits moist moisture feeling, refreshing feeling of use, excellent long-term water resistance, and excellent UV-blocking effect, despite including a small amount of UV-blocking components.

The cosmetic composition may be prepared in a formulation including a skin lotion, a skin softener, a skin toner, an astringent, a lotion, a milk lotion, a moisturizing lotion, a nourishing lotion, a massage cream, a nourishing cream, a moisturizing cream, an eye cream, a hand cream, a foundation, an essence, a nourishing essence, an eye essence, a pack, a soap, a cleansing foam, a cleansing lotion, a cleansing cream, cleansing water, a cleansing oil, a cleansing tissue, a hand sanitizer, a body lotion, a body cream, a body cleanser, a suspension, a gel, a powder, a paste, a shampoo, a hair essence, a treatment, a rinse, a scalp pack, a mask pack, or a sheet or aerosol composition. Compositions of such formulations may be prepared according to typical methods in the art.

The components included in the cosmetic composition may include components commonly used in cosmetic compositions, and may include, for example, typical auxiliary agents and carriers such as stabilizers, solubilizers, vitamins, pigments, and fragrances.

Another aspect provides a use of a composition including a film former and an UV-blocking agent for the preparation of a sunscreen cosmetic composition. In the above use, the same terms or elements mentioned above as those already mentioned are as above.

Another aspect provides a method of protecting the skin of an individual from UV radiation, including applying an effective amount of a composition including a film former and an UV-blocking agent to the individual. In the above method, the same terms or elements mentioned above as those already mentioned are as above.

The term "applying" may refer to placing a composition according to an embodiment into an individual by a method or route that results in at least partial localization of the composition to a desired site according to an embodiment. The application may include application to the skin.

The term "effective amount" refers to an amount effective enough to exhibit the effect mentioned above.

The individual may be a mammal, for example a human. The individual may be an individual requiring UV-blocking effect.

### Advantageous Effects of Invention

The composition according to an aspect is an oil-in-water type sunscreen cosmetic composition including hydrogenated castor oil/sebacic acid copolymer as a film former, which, despite including a small amount of ultraviolet (UV)-blocking components, exhibits moist moisture feeling, refreshing feeling of use, and excellent long-term water resistance, while also having excellent UV-blocking effect, and thus may be usefully employed as an oil-in-water type waterproof sunscreen cosmetic composition.

### Mode for the Invention

The present disclosure will be explained in more detail in the following examples. However, these examples are intended to illustrate one or more embodiments by way of example and the scope of the present disclosure is not limited to these examples.

### [Example]

### Example 1 to 11. Preparation of Oil-in-Water Type Sunscreen Cosmetic Composition

An oil-in-water type sunscreen cosmetic composition was prepared as follows. The specific components and contents of the composition are as shown in Table 1 below.

Specifically, an aqueous phase portion and a thickener were placed in a stirrer and stirred at 5000 rpm for about 5 to 10 minutes, followed by heating to 80 °C. Here, an oil phase portion was also heated to 80 °C, and after confirming complete dissolution, it was slowly added while stirring with the stirrer at 3000 rpm. After the addition was completed, the stirring speed was increased to 8000 rpm and stirring was continued for about 10 minutes, followed by cooling to 30 °C to finally obtain the oil-in-water type sunscreen cosmetic compositions of Examples 1 to 11.

**[Table 1]**

| Ph as e CI as sifi cat ion | Component | Ex am ple 1 | Ex am ple 2 | Ex am ple 3 | Ex am ple 4 | Ex am ple 5 | Ex am ple 6 | Ex am ple 7 | Ex am ple 8 | Ex am ple 9 | Ex am ple 10 | Ex am ple 11 |
|---|---|---|---|---|---|---|---|---|---|---|---|---|
| Aq ue ou s Ph as e Po rti on | Purified water | To 10 0 | To 10 0 | To 10 0 | To 10 0 | To 10 0 | To 10 0 | To 10 0 | To 10 0 | To 10 0 | To 10 0 | To 10 0 |
| | Propanediol | 5.0 0 | 5.0 0 | 5.0 0 | 5.0 0 | 5.0 0 | 5.0 0 | 5.0 0 | 5.0 0 | 5.0 0 | 5.0 0 | 5.0 0 |
| | 1,2-Hexanediol | 0.8 0 | 0.8 0 | 0.8 0 | 0.8 0 | 0.8 0 | 0.8 0 | 0.8 0 | 0.8 0 | 0.8 0 | 0.8 0 | 0.8 0 |
| | Pentylene glycol | 0.9 0 | 0.9 0 | 0.9 0 | 0.9 0 | 0.9 0 | 0.9 0 | 0.9 0 | 0.9 0 | 0.9 0 | 0.9 0 | 0.9 0 |
| | Hydroxyacetophenone | 0.3 0 | 0.3 0 | 0.3 0 | 0.3 0 | 0.3 0 | 0.3 0 | 0.3 0 | 0.3 0 | 0.3 0 | 0.3 0 | 0.3 0 |
| | Sodium stearoyl glutamate | 0.1 0 | 0.1 0 | 0.1 0 | 0.1 0 | 0.1 0 | 0.1 0 | 0.1 0 | 0.1 0 | 0.1 0 | 0.1 0 | 0.1 0 |
| Thickener | Carbomer | 0.1 2 | 0.1 2 | 0.1 2 | 0.1 2 | 0.1 2 | 0.1 2 | 0.1 2 | 0.1 2 | 0.1 2 | 0.1 2 | 0.1 2 |
| | Sodium acrylate crosspolymer-2 | 0.0 3 | 0.0 3 | 0.0 3 | 0.0 3 | 0.0 3 | 0.0 3 | 0.0 3 | 0.0 3 | 0.0 3 | 0.0 3 | 0.0 3 |
| | Acrylate/C10-30 alkyl acrylate crosspolymer | 0.1 8 | 0.1 8 | 0.1 8 | 0.1 8 | 0.1 8 | 0.1 8 | 0.1 8 | 0.1 8 | 0.1 8 | 0.1 8 | 0.1 8 |
| | Polyacrylate crosspolymer-6 | 0.1 0 | 0.1 0 | 0.1 0 | 0.1 0 | 0.1 0 | 0.1 0 | 0.1 0 | 0.1 0 | 0.1 0 | 0.1 0 | 0.1 0 |
| Oil Phase Portion | Polyglyceryl-3 distearate | 2.0 0 | 2.0 0 | 2.0 0 | 2.0 0 | 2.0 0 | 2.0 0 | 2.0 0 | 2.0 0 | 2.0 0 | 2.0 0 | 2.0 0 |
| | Hydrogenated castor oil/sebacic acid copolymer | 0.5 0 | 1.0 0 | 2.0 0 | 0.5 0 | 1.0 0 | 2.0 0 | 0.5 0 | 1.0 0 | 2.0 0 | 1.5 0 | 1.5 0 |
| | Diisopropyl sebacate | 20. 00 | 20. 00 | 20. 00 | 20. 00 | 20. 00 | 20. 00 | 20. 00 | 20. 00 | 20. 00 | 20. 00 | 20. 00 |
| | Diethylaminohydroxybe nzoylhexyl benzoate | 0.5 0 | 0.5 0 | 0.5 0 | 5.0 0 | 5.0 0 | 5.0 0 | 10. 00 | 10. 00 | 10. 00 | - | 5.0 0 |
| | Ethylhexyltriazone | 0.5 0 | 0.5 0 | 0.5 0 | 3.0 0 | 3.0 0 | 3.0 0 | 5.0 0 | 5.0 0 | 5.0 0 | 3.0 0 | - |
| | Glyceryl stearate | 0.5 0 | 0.5 0 | 0.5 0 | 0.5 0 | 0.5 0 | 0.5 0 | 0.5 0 | 0.5 0 | 0.5 0 | 0.5 0 | 0.5 0 |
| | Behenyl alcohol | 0.5 0 | 0.5 0 | 0.5 0 | 0.5 0 | 0.5 0 | 0.5 0 | 0.5 0 | 0.5 0 | 0.5 0 | 0.5 0 | 0.5 0 |

### [Comparative Example]

### Comparative Example 1. Preparation of a Composition Not Including Hydrogenated Castor Oil/Sebacic Acid Copolymer

Comparative Example 1 was prepared in the same manner as Example 1, except that the film former of Examples 1 to 11, hydrogenated castor oil/sebacic acid copolymer, was not included. The specific components and contents of the composition are as shown in Table 2 below.

**[Table 2]**

| Phase Classification | Component | Comparative Example 1 | Comparative Example 2 | Comparative Example 3 |
|---|---|---|---|---|
| Aqueous Phase Portion | Purified water | To 100 | To 100 | To 100 |
| | Propanediol | 5.00 | 5.00 | 5.00 |
| | 1,2-Hexanediol | 0.80 | 0.80 | 0.80 |
| | Pentylene glycol | 0.90 | 0.90 | 0.90 |
| | Hydroxyacetophenone | 0.30 | 0.30 | 0.30 |
| | Sodium stearoyl glutamate | 0.10 | 0.10 | 0.10 |
| Thickener | Carbomer | 0.12 | 0.12 | 0.12 |
| | Sodium acrylate crosspolymer-2 | 0.03 | 0.03 | 0.03 |
| | Acrylate/C10-30 alkyl acrylate crosspolymer | 0.18 | 0.18 | 0.18 |
| | Polyacrylate crosspolymer-6 | 0.10 | 0.10 | 0.10 |
| Oil Phase Portion | Polyglyceryl-3 distearate | 2.00 | 2.00 | 2.00 |
| | Hydrogenated castor oil/sebacic acid copolymer | X | X | X |
| | Diisopropyl sebacate | 20.00 | 20.00 | 20.00 |
| | Diethylaminohydroxybenzoylhexyl benzoate | 0.50 | 5.00 | 10.00 |
| | Ethylhexyltriazone | 0.50 | 3.00 | 5.00 |
| | Glyceryl stearate | 0.50 | 0.50 | 0.50 |
| | Behenyl alcohol | 0.50 | 0.50 | 0.50 |

### [Experimental Example]

### Experimental Example 1. Evaluation of Ultraviolet Blocking Effect

### 1-1. Evaluation by Measurement of SPF Value

To evaluate the UVB ultraviolet (UV)-blocking effect of the Examples and Comparative Examples, an experiment for measuring the Sun Protection Factor (SPF) value was conducted as follows.

Specifically, the UV transmittance measurement was performed by reference to the test specifications described in the "method for the in-vitro determination of UVA protection provided by sunscreen products" of European Cosmetics Association (COLIPA), and a sample was applied to a polymethyl methacrylate (PMMA HELIO PLATE HD6) plate, and measurement was conducted using UV-2000S (Labsphere, Inc. USA). The application amount of the sample was set to 28.6 mg (1.3 mg/cm² × 22 cm²) with reference to the amount mentioned in COLIPA, and after drying in a dark room for about 15 minutes, the measurement was conducted.

The transmittance measurement wavelength range was measured in the range of 290 nm to 400 nm using UV-2000S, and the SPF value was calculated using LABSPHERE. A higher SPF index (sun protection factor) indicates more effective UV blocking, and the SPF index was calculated according to the Equation 1 below. [Equation 1] $SPF = \frac{{\int }_{290}^{400} S \left(λ\right) {s}_{er} \left(λ\right) dλ}{{\int }_{290}^{400} S \left(λ\right) {s}_{er} \left(λ\right) T \left(λ\right) dλ}$

As a result, as shown in Table 3 below, it may be seen that the UVB UV-blocking effect is improved in Examples 1 to 9 including a hydrogenated castor oil/sebacic acid copolymer as a film former, compared to Comparative Examples 1 to 3 not including a film former. Specifically, Examples 1 to 3, which include the same amount of UV-blocking component as Comparative Example 1, showed an improvement in UV-blocking effect by about 2.5 to 4 times depending on the content of the film former, Examples 4 to 6, which include the same amount of UV-blocking component as Comparative Example 2, showed an improvement in UV-blocking effect by about 1.3 to 2.9 times depending on the content of the film former, and Examples 7 to 9, which include the same amount of UV-blocking component as Comparative Example 3, showed an improvement in UV-blocking effect by about 1.9 to about 2.3 times depending on the content of the film former. In particular, it may be seen that Examples 6 to 9 have remarkably excellent UV-blocking effects.

**[Table 3]**

| | SPF (IN-VITRO) |
|---|---|
| Example 1 | 5.3 |
| Example 2 | 7.5 |
| Example 3 | 8.5 |
| Example 4 | 16.7 |
| Example 5 | 23.9 |
| Example 6 | 35.6 |
| Example 7 | 54.2 |
| Example 8 | 58.7 |
| Example 9 | 64.5 |
| Comparative Example 1 | 2.1 |
| Comparative Example 2 | 12.3 |
| Comparative Example 3 | 28 |

### 1-2. Evaluation by Measurement of PA Value

To evaluate the UVA UV-blocking effect of the Examples and Comparative Examples, an experiment for measuring the Protection Grade of UVA (PA) value was conducted as follows.

Specifically, the UVA protection factor (UVAPF) was measured using UV-2000S (Labsphere, Inc. USA). The standard referred to the "Method for in vitro determination of UVA protection, 2009" for the in vitro evaluation of UVA protection, and the application amount of the sample was set to 28.6 mg (1.3 mg/cm² × 22 cm²) with reference to the amount mentioned in European Cosmetics Association (COLIPA), and after drying for about 30 minutes, the measurement was conducted.

As a result, as shown in Table 4 below, it may be seen that the UVA UV-blocking effect is improved in Examples including a hydrogenated castor oil/sebacic acid copolymer as a film former, compared to Comparative Examples not including a film former. Specifically, Examples 4 to 6, which include the same amount of UV-blocking component as Comparative Example 2, confirmed improvement in UV-blocking effect depending on the content of the film former. In addition, Example 11, which includes the same amount of diethylaminohydroxybenzoylhexyl benzoate as Comparative Example 2, was confirmed to have an improved UV-blocking effect due to the inclusion of hydrogenated castor oil/sebacic acid copolymer, despite having a lower content of UV-blocking components. In particular, it may be seen that examples 5 to 9 and 11 have remarkably excellent UV ray blocking effects.

**[Table 4]**

| | PA (IN-VITRO) |
|---|---|
| Example 1 | + |
| Example 2 | + |
| Example 3 | + |
| Example 4 | +++ |
| Example 5 | ++++ |
| Example 6 | ++++ |
| Example 7 | ++++ |
| Example 8 | ++++ |
| Example 9 | ++++ |
| Example 11 | ++++ |
| Comparative Example 1 | + |
| Comparative Example 2 | +++ |
| Comparative Example 3 | ++++ |

### Experimental Example 2. Evaluation of Long-term water resistance

To evaluate the long-term water resistance of the Examples and Comparative Examples, an experiment was conducted to measure the SPF values before and after the long-term water resistance test, as follows.

For the water-resistant UV-blocking transmittance, the sample was applied to the HD-6 plate, left for 20 minutes, and attached to the bottom of a 2 L beaker. Afterwards, 1000 ml of tap water at 40 °C was added, and the mixture was stirred for 40 minutes at a rotation speed of 250 rpm using an AGI mixer, dried for 30 minutes, and the SPF values before and after the long-term water resistance test were compared and measured.

The measurement of SPF values was conducted in the same manner as described in the UV-blocking ability evaluation of Experimental Example 1-1.

As a result, as shown in Table 5 below, it may be seen that the long-term water resistance is improved in Examples 1 to 9 including a hydrogenated castor oil/sebacic acid copolymer as a film former, compared to Comparative Examples 1 to 3 not including a film former. Specifically, Examples 1 to 3, which include the same amount of UV-blocking component as Comparative Example 1, showed improved continuous water resistance depending on the content of the film former, unlike Comparative Example 1, which could not measure water resistance, Examples 4 to 6, which include the same amount of UV-blocking component as Comparative Example 2, showed improved long-term water resistance by about 2.9 to 7.9 times depending on the content of the film former, and Examples 7 to 9, which include the same amount of UV-blocking component as Comparative Example 3, showed improved long-term water resistance by about 4.6 to about 5.8 times depending on the content of the film former. In addition, Example 10, which includes the same amount of ethylhexyl triazone as Comparative Example 2, was confirmed to have improved long-term water resistance due to the inclusion of hydrogenated castor oil/sebacic acid copolymer, despite having a lower content of UV-blocking components.

**[Table 5]**

| | Water Resistance SPF (IN-VITRO) |
|---|---|
| Example 1 | 3.7 |
| Example 2 | 5.5 |
| Example 3 | 6.1 |
| Example 4 | 10.1 |
| Example 5 | 14.3 |
| Example 6 | 27.6 |
| Example 7 | 34 |
| Example 8 | 40 |
| Example 9 | 42.3 |
| Example 10 | 4.3 |
| Comparative Example 1 | Not measurable |
| Comparative Example 2 | 3.5 |
| Comparative Example 3 | 7.4 |

### Experimental Example 3. Evaluation of Feeling of Use and Spreadability

To evaluate the feeling of use and spreadability of the Examples and Comparative Examples, a sensory evaluation was conducted as follows.

Specifically, the feeling of use and spreadability evaluation was conducted by evaluating the feeling of use and spreadability of the cosmetic composition by a skin panel test. Each sample was applied to the skin and rolled, and the feeling of use and spreadability at the point of change in phase were tested by 10 panel testers, and the panel evaluated them on a scale of 1 to 5, and the mean value of the scores evaluated by the 10 panel testers was calculated to confirm the feeling of use and spreadability scores.

The evaluation scale was as follows: 5 points - very good, 4 points - good, 3 points - average, 2 points - poor, 1 point - very poor.

As a result, it may be seen that the feeling of use and spreadability are improved in Examples 1 to 9 including a hydrogenated castor oil/sebacic acid copolymer as a film former compared to Comparative Examples 1 to 3 that do not include a film former. Specifically, Examples 1 to 3, which include the same amount of UV-blocking component as Comparative Example 1, showed an improvement in feeling of use and spreadability by 5 points depending on the content of the film former, Examples 4 to 6, which include the same amount of UV-blocking component as Comparative Example 2, showed an improvement in feeling of use and spreadability by 4 or 5 points depending on the content of the film former, and Examples 7 to 9, which include the same amount of UV-blocking component as Comparative Example 3, showed an improvement in feeling of use and spreadability by 3 or 4 points depending on the content of the film former. In addition, Example 10, which includes the same amount of ethylhexyl triazone as Comparative Example 2, and Example 11, which includes the same amount of diethylaminohydroxybenzoylhexyl benzoate, were confirmed to have improved feeling of use and spreadability due to the inclusion of hydrogenated castor oil/sebacic acid copolymer.

That is, when a hydrogenated castor oil/sebacic acid copolymer is included as a film former, it may be seen that the UV-blocking effect and long-term water resistance are improved, and at the same time, the feeling of use and spreadability are improved.

**[Table 6]**

| | Sensory Evaluation |
|---|---|
| Example 1 | 5 |
| Example 2 | 5 |
| Example 3 | 5 |
| Example 4 | 5 |
| Example 5 | 5 |
| Example 6 | 4 |
| Example 7 | 4 |
| Example 8 | 4 |
| Example 9 | 3 |
| Example 10 | 4 |
| Example 11 | 4 |
| Comparative Example 1 | 4 |
| Comparative Example 2 | 2 |
| Comparative Example 3 | 1 |

In summary, it was confirmed that a sunscreen cosmetic composition including a hydrogenated castor oil/sebacic acid copolymer according to an aspect has a moist moisture feeling, a refreshing feeling of use, and excellent long-term water resistance, and at the same time, has an excellent UV-blocking effect, despite using a small amount of a UV-blocking component, and may be used as an oil-in-water type waterproof sunscreen cosmetic composition.

## Claims

1. A sunscreen cosmetic composition comprising a film former and an ultraviolet-blocking agent.

2. The composition of claim 1, wherein the composition is an oil-in-water (O/W) type.

3. The composition of claim 1, wherein the film former comprises a hydrogenated castor oil/sebacic acid copolymer.

4. The composition of claim 3, wherein the hydrogenated castor oil/sebacic acid copolymer comprises 0.01 to 10 wt% based on the total weight of the composition.

5. The composition of claim 1, wherein the ultraviolet-blocking agent comprises one or more selected from the group consisting of ethylhexyl triazone and diethylamino hydroxybenzoyl hexyl benzoate.

6. The composition of claim 5, wherein the ethylhexyl triazone is comprised in an amount of 0.05 to 20 wt% based on the total weight of the composition.

7. The composition of claim 5, wherein the diethylamino hydroxybenzoyl hexyl benzoate is comprised in an amount of 0.05 to 30 wt% based on the total weight of the composition.

8. The composition of claim 1, wherein the composition comprises the film former and the ultraviolet-blocking agent in a weight ratio of 1:0.1 to 50.

9. A use of a composition comprising a film former and an ultraviolet-blocking agent, for the preparation of a sunscreen cosmetic composition.

10. A method of protecting the skin of an individual from ultraviolet radiation, comprising applying an effective amount of a composition including a film former and an ultraviolet-blocking agent to the individual.
